(19) Europäisches Patentamt
European Patent Office
Office européen des brevets

(11) Veröffentlichungsnummer : **0 537 534 B1**

# (12) EUROPÄISCHE PATENTSCHRIFT

(45) Veröffentlichungstag der Patentschrift : **22.06.94 Patentblatt 94/25**

(51) Int. Cl.$^5$ : **C07D 211/22,** C07C 271/16, // A01N47/12, A01N47/16

(21) Anmeldenummer : **92116522.1**

(22) Anmeldetag : **28.09.92**

## (54) Verfahren zur Herstellung von N-(Hydroxyalkyl)-carbamidsäure- alkylestern.

(30) Priorität : **10.10.91 DE 4133516**

(43) Veröffentlichungstag der Anmeldung : **21.04.93 Patentblatt 93/16**

(45) Bekanntmachung des Hinweises auf die Patenterteilung : **22.06.94 Patentblatt 94/25**

(84) Benannte Vertragsstaaten : **BE CH DE ES FR GB IT LI NL**

(56) Entgegenhaltungen :
**EP-A- 0 289 842**
**DE-A- 1 150 973**
**DE-A- 3 239 390**

(56) Entgegenhaltungen :
**JOURNAL OF THE AMERICAN CHEMICAL SOCIETY Bd. 69, 1947, Seiten 1384 - 1387;'Synthesis of Cephalin'**
**J. CHEM. SOC. PERKIN TRANS I 1988, Seiten 2251 - 2253; 'Cycloaddition of 2,3,4,5-tetrahydropyridine N-Oxide to isobutyl vinyl ether and ally alcohol; Methyl2-formylmethylpiperidine-1-carboxylate'**

(73) Patentinhaber : **BAYER AG**
**D-51368 Leverkusen (DE)**

(72) Erfinder : **Krebs, Andreas, Dr.**
**Am Gartenfeld 70**
**W-5068 Odenthal-Holz (DE)**
Erfinder : **Krüger, Bernd-Wieland, Dr.**
**Unterboschbach 19**
**W-5060 Bergisch Gladbach 2 (DE)**

**EP 0 537 534 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von bekannten N-(Hydroxyalkyl)-carbamidsäure-alkylestern.

Es ist bereits bekannt, daß N-(Hydroxyalkyl)-carbamidsäure-alkylester durch Reaktion von Aminoalkoholen mit Chlorameisensäureestern erhalten werden können. Diese Reaktion wird üblicherweise in einem inerten organischen Lösungsmittel in Gegenwart eines Säureakzeptors bei Temperaturen von -20°C bis +20°C durchgeführt. Beispielhaft seien die Umsetzung von 2-Methylamino-ethanol mit Chlorameisensäure-benzylester und Triethylamin in Chloroform bei 20°C (DE-A 3 239 390), von 2-(2-Hydroxyethyl)-piperidin mit Chlorameisensäure-butylester und Triethylamin in Tetrahydrofuran bei -20°C (EP-A 289 842) und die Umsetzung von 2,2-Diethyl-3-hydroxy-propylamin mit Chlorameisensäure-ethylester und Triethylamin in Benzol unter Kühlung, gefolgt von 2,5-stündigem Erhitzen unter Rückfluß (DE-PS 1 150 973), genannt. Außerdem ist bekannt, daß 2-(2-Hydroxyethyl)-piperidin mit Chlorameisensäuremethylester bei 0 bis 20°C in Gegenwart einer wäßrigen Natriumhydrogencarbonat-Lösung zu 2-(2-Hydroxyethyl)-piperidinyl-carbamidsäure-methylester umgesetzt werden kann (J. Chem. Soc. Perkin Trans. I 1988, 2251 bis 2253). Weiterhin ist bekannt, daß 2-Aminoethanol mit Chlorameisensäure-benzylester bei 0°C unter Verwendung von 4N Natronlauge als Säureakzeptor zu N-(2-Hydroxyethyl)-carbamidsäure-benzylester umgesetzt werden kann [J. Am. Chem. Soc. 69, 1384 (1947)].

Die genannten Verfahren weisen in der Regel mehrere Nachteile auf, die eine Umsetzung in den technischen Maßstab erschweren oder unmöglich machen: Die Rohprodukte sind häufig verunreinigt und müssen durch Destillation oder durch Chromatographie gereinigt werden, was neben dem erhöhten Aufwand auch zu beträchtlichen Ausbeuteverlusten führen kann. Als Folge der Hauptnebenreaktion werden Produkte gebildet, die sich aus einer konkurrierenden Reaktion des Chlorameisensäureesters mit der Hydroxy-Gruppe ableiten. Bei der destillativen Reinigung können auch bei niedrigen Drucken Zersetzungsreaktionen auftreten, deren übelriechende Produkte das Destillat verunreinigen. Außerdem hat die Verwendung von tertiären Aminen als Säureakzeptoren einen erhöhten Aufwand bei der Aufbereitung oder der Entsorgung der dabei anfallenden Amin-Hydrochloride zur Folge. Reaktionstemperaturen von 0 bis 20°C führen unter technischen Bedingungen zu langen Reaktionszeiten, damit die Wärme der stark exothermen Reaktion abgeführt werden kann.

Es bestand also Bedarf nach einem einfach durchzuführenden Verfahren zur Umsetzung von Chlorameisensäureestern mit N-(Hydroxyalkyl)-aminen, das in hohen Ausbeuten und sehr guten Raum-Zeit-Ausbeuten und ohne problematische Aufarbeitung zu ausreichend reinen Produkten führt.

Es wurde nun gefunden, daß man N-(Hydroxyalkyl)-carbamidsäurealkylester der Formel (I)

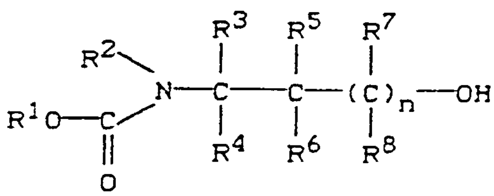

(I)

in welcher $R^1$ für geradkettiges oder verzweigtes Alkyl mit 1 bis 12 Kohlenstoffatomen steht und $R^2$ bis $R^8$ gleich oder verschieden sind und für Wasserstoff oder für Alkylreste mit 1 bis 8 Kohlenstoffatomen stehen oder $R^2$ und $R^3$ gemeinsam mit den Atomen, an welche sie gebunden sind, einen Ring mit 5 bis 7 Atomen, die mit $C_1$-$C_4$-Alkylresten substituiert sein können bilden, und n 0 oder 1 bedeutet
erhalten werden können, wenn man Aminoalkohole der Formel (II)

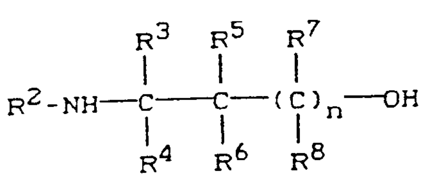

(II)

in welcher $R^2$ bis $R^8$ und n die oben angegebene Bedeutung haben,
mit Chlorameisensäureestern der Formel (III)

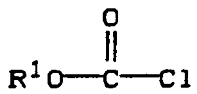

(III)

in welcher $R^1$ die oben angegebene Bedeutung hat
in Gegenwart von wäßrigen Alkalimetall-hydroxid-Lösungen und gegebenenfalls in Anwesenheit eines organischen Lösungsmittels bei Temperaturen zwischen 30°C und 110°C umsetzt.

Das erfindungsgemäße Verfahren weist eine Reihe von Vorteilen auf: so sind die Ausbeuten sehr hoch und erreichen 80 bis über 90 % der Theorie. Die Raum-Zeit-Ausbeuten sind ebenfalls sehr gut, da die Umsetzung mit wenig oder auch ohne Verdünnungsmittel und mit relativ konzentrierten Säureakzeptor-Lösungen durchgeführt werden kann. Außerdem werden bei dem Verfahren wenig Nebenprodukte gebildet, so daß nach einfachen Aufarbeitungsschritten Produkt-Reinheiten von über 98 % erzielt werden. Schließlich erlauben die Reaktionstemperaturen von 30 bis 110°C eine Kühlung des Reaktors mit Wasser.

Es ist als ausgesprochen überraschend zu bezeichnen, daß gemäß dem erfindungsgemäßen Verfahren so gute Ausbeuten erzielt werden, denn es ist allgemein bekannt, daß Chlorameisensäureester durch Wasser und insbesondere durch wäßrige Alkalimetallhydroxid-Lösungen rasch hydrolysiert werden. Dies trifft besonders bei erhohten Reaktionstemperaturen zu. Außerdem war es völlig unerwartet, daß bei erhohten Reaktionstemperaturen reinere Reaktionsprodukte erhalten werden als bei tieferen Reaktionstemperaturen (vergl. z.B.: R.T. Morrison und R.N. Boyd, Lehrbuch der organischen Chemie, S. 128, 3. Auflage, Verlag Chemie, Weinheim, 1986: "Es ist eine allgemeine Tatsache, daß mit einer Erhöhung der Temperatur die Selektivität eines Reagenzes hinsichtlich der Angriffs-Position abnimmt, umgekehrt wächst die Selektivität mit sinkender Temperatur.")

Verwendet man zum Beispiel 2-(2-Hydroxyethyl)-piperidin und Chlorameisensäure-sec.-butylester als Ausgangsstoffe, so kann der Reaktionsablauf durch das folgende Formelschema wiedergegeben werden:

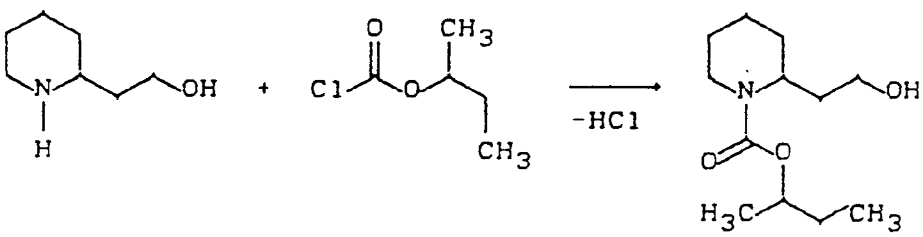


Besonders bevorzugt sind Verbindungen der Formeln (I) und (II), bei denen die Reste $R^2$ bis $R^8$ für Wasserstoff, Methyl oder Ethyl stehen, oder die Reste $R^2$ und $R^3$, gemeinsam mit den Atomen, an welche sie gebunden sind, einen unsubstituierten Ring mit 5 bis 7 Atomen bilden, und bei denen n 0 oder 1 bedeutet.

Ganz besonders bevorzugt sind Verbindungen der Formeln (I) und (II), bei denen $R^2$ Methyl und $R^3$ bis $R^8$ Wasserstoff oder bei denen die Reste $R^2$ und $R^3$, gemeinsam mit den Atomen, an welche sie gebunden sind, den Piperidin-Ring bedeuten, und n 1 bedeutet.

Die Verbindungen der Formel (II) sind bereits bekannt (vergl. z.B. Cesare Ferri, Reaktionen der org. Synthese, Georg Thieme Verlag Stuttgart, 1978, S. 211 ff und 496 bis 497).

Als Beispiele für die Ausgangsstoffe der Formel (II) seien genannt:
2-Amino-ethanol
2-Methylamino-ethanol
2-Ethylamino-ethanol
3-Amino-propanol
3-Methylamino-propanol
1-Amino-2-propanol
2-Amino-1-butanol
2-Amino-2-methyl-propanol
2-(2-Hydroxyethyl)-piperidin
2-Hydroxymethyl-piperidin
2-Hydroxymethyl-pyrrolidin
2-(2-Hydroxy-ethyl)-pyrrolidin

Besonders bevorzugt sind Verbindungen der Formel (III), bei denen $R^1$ für einen geradkettigen oder verzweigten Alkylrest mit 1 bis 5 Kohlenstoffatomen steht.

Als Beispiele für die Verbindungen der Formel (III) seien genannt:
Chlorameisensäure-methylester
Chlorameisensäure-ethylester
Chlorameisensäure-(1-methyl-ethyl)-ester
Chlorameisensäure-propylester
Chlorameisensäure-(1-methyl-propyl)-ester
Chlorameisensäure-(2-methyl-propyl)-ester
Chlorameisensäure-(2,2-dimethyl-propyl)-ester
Chlorameisensäure-butylester
Chlorameisensäure-(1-methyl-butyl)-ester
Chlorameisensäure-(2-methyl-butyl)-ester
Chlorameisensäure-(3-methyl-butyl)-ester
Chlorameisensäure-(1,3-dimethyl-butyl)-ester
Chlorameisensäure-(3,3-dimethyl-butyl)-ester
Chlorameisensäure-(2-ethyl-butyl)-ester
Chlorameisensäure-pentylester
Chlorameisensäure-(2-methyl-pentyl)-ester
Chlorameisensäure-hexylester
Chlorameisensäure-(2-ethyl-hexyl)-ester

Die erfindungsgemäß herstellbaren N-(Hydroxyalkyl)-carbamidsäurealkylester der Formel (I) sind beispielsweise aus EP-A 289 842 bekannt.

Als Verdünnungsmittel für das erfindungsgemäße Verfahren kommen alle inerten organischen Lösungsmittel in Frage. Hierzu gehören vorzugsweise Kohlenwasserstoffe wie Petrolether, Ligroin, Hexan, Cyclohexan, Methylcyclohexan, Toluol und Xylol, Ether wie Diethylether, Diisopropylether, Methyl-tert.-butyl-ether, Methyl-tert.-amyl-ether und Anisol sowie halogenierte Kohlenwasserstoffe wie Dichlormethan, 1,1,2-Trifluortrichlor-ethan, Chlorbenzol und 1,2-Dichlorbenzol und deren Gemische. Die Reaktion kann aber auch ohne Lösungsmittel oder unter Verwendung des Reaktionsproduktes oder eines Nebenproduktes aus dem Chlorameisensäureester, dem entsprechenden Kohlensäureester, als Verdünnungsmittel durchgeführt werden.

Die erfindungsgemäße Umsetzung wird in Gegenwart von wäßrigen Alkalimetall-hydroxid-Lösungen als Säureakzeptoren durchgeführt. Als Beispiele seien Lithiumhydroxid, Natriumhydroxid und Kaliumhydroxid genannt. Vorzugsweise wird eine wäßrige Natriumhydroxid-Lösung eingesetzt.

Die Umsetzung wird bei Temperaturen zwischen 30 und 110°C, vorzugsweise zwischen 50 und 90°C, vorgenommen.

Die Umsetzung kann bei Normaldruck, aber auch bei erhöhtem oder bei erniedrigtem Druck vorgenommen werden. Im allgemeinen arbeitet man bei Normaldruck.

Bei der Durchführung des erfindungsgemäßen Verfahrens setzt man auf 1 Mol des Ausgangsmaterials der Formel (II) 0,6 bis 1,3 Mol des Reaktanden der Formel (III) ein. Bevorzugt ist eine Umsetzung von 1 Mol des Ausgangsmaterials der Formel (II) mit 0,8 bis 1,0 Mol des Reaktanden der Formel (III). Das Alkalimetallhydroxid wird in Mengen von 0,9 bis 2,0 Mol, bezogen auf 1 Mol des Reaktanden der Formel (III), eingesetzt. Bevorzugt werden 1,0 bis 1,2 Mol Alkalimetallhydroxid verwendet.

Bei der Durchführung des erfindungsgemäßen Verfahrens sind mehrere Arten der Reaktionsführung möglich. Beispielsweise wird das Ausgangsmaterial der Formel (II) mit der wäßrigen Alkalimetallhydroxid-Lösung vorgelegt und der Reaktand der Formel (III) zudosiert. Man kann aber auch das Ausgangsmaterial der Formel (II), gegebenenfalls auch als wäßrige Lösung, vorlegen und simultan den Reaktanden der Formel (III) und die Alkalimetallhydroxid-Lösung zudosieren, oder die Alkalimetallhydroxid-Lösung vorlegen und die beiden Reaktionspartner simultan zudosieren. Außerdem ist es möglich, die beiden Reaktanden und die Alkalimetallhydroxid-Lösung gleichzeitig in einen Reaktor einzudosieren.

Als Reaktionsgefäße eignen sich die üblichen Reaktoren, beispielsweise Reaktionskessel, Reaktionskessel-Kaskaden und Rohrreaktoren.

Die erfindungsgemäße Reaktions-Temperatur des Verfahrens kann auf mehreren Wegen erreicht werden, beispielsweise durch Vorerwärmen des Reaktanden der Formel (II) und der Alkalimetallhydroxid-Lösung und/oder durch Ausnutzen der Reaktionswärme.

Die Aufarbeitung erfolgt nach üblichen Methoden, beispielsweise durch Abtrennen der wäßrigen Phase und Waschen der organischen Phase mit verdünnten Mineralsäuren und Wasser, wobei die organische Phase gegebenenfalls mit einem mit Wasser nicht mischbaren Lösungsmittel verdünnt werden kann. Leichter flüchtige Verunreinigungen lassen sich durch sogenanntes "Andestillieren", d.h. durch längeres Erhitzen unter vermindertem Druck auf mäßig erhöhte Temperatur, entfernen, wobei gegebenenfalls Wasser zugesetzt oder Wasserdampf eingeblasen werden kann, um die Abtrennung wasserdampfflüchtiger Verunreinigungen zu erleichtern.

Die erfindungsgemäß hergestellten Stoffe können als Mittel zur Insekten- und Milbenabwehr eingesetzt werden (vergl. EP-A 289 842).

## Herstellungsbeispiele

### Beispiel 1

2-(2-Hydroxyethyl)-piperidyl-carbamidsäure-sec.-butylester

In einem 1,0 l Reaktionsgefäß mit Bodenauslaß-Ventil werden 258,4 g (2 mol) 2-(2-Hydroxyethyl)-piperidin bei 60°C in 249 g Wasser gelöst, 195,5 g (2,2 mol) 45 %ige Natronlauge zugesetzt und bei 60°C Innentemperatur unter kräftigem Rühren 259,4 g (1,9 mol) Chlorameisensäure-1-methyl-propylester so zugetropft, daß die Innentemperatur 80°C erreicht (Dauer 40 min). Dann wird die Innentemperatur auf 25°C abgesenkt, die wäßrige Phase abgetrennt und die organische Phase mit 340 ml Methylcyclohexan versetzt. Es wird zwei mal mit je 120 ml 1N Schwefelsäure jeweils 5 min ausgerührt, dann noch viermal mit je 120 ml Wasser neutral gewaschen und das Methylcyclohexan bei 18 mbar bis zu einer Innentemperatur von 80°C abdestilliert in eine tiefgekühlte Vorlage. Man läßt unter Vakuum auf 30°C erkalten, setzt 20 ml Wasser zu, rührt bis zur Homogenisierung und destilliert bei 30°C/18 mbar ab. Dieser Vorgang wird noch einmal wiederholt, wobei bis 60°C Innentemperatur andestilliert wird.
Ausbeute: 408,1 g
Gehalt: 99,2 %
(= 92,9 % der Theorie, bezogen auf den Chlorameisensäureester)

### Beispiel 2

2-(2-Hydroxyethyl)-piperidinyl-carbamidsäure-sek.-butylester

In einem 20 l Reaktionsgefäß mit Bodenauslaßventil werden 2 192 g (24,66 mol) 45 %ige Natronlauge vorgelegt und 2 896 g 2-(2-Hydroxyethyl)-piperidin (Gehalt nach Perchlorsäure-Titration 98,2 %, = 22 mol) als Lösung in 2 800 g Wasser zugegeben.

Beginnend bei 50°C Innentemperatur und 50°C Manteltemperatur werden dann 2 907 g Chlorameisensäure-sek.-butylester (97,5 %ig, = 20,75 mol) unter Rühren innerhalb von 60 min zudosiert, wobei nach ca. 1 000 g und 20 min die Innentemperatur 80°C erreicht, worauf die Manteltemperatur auf 30°C gesenkt wird, um eine Innentemperatur von 80 bis 85°C zu halten. Dann wird innerhalb von 1 Stunde unter Rühren auf 20°C abgekühlt und die wäßrige Phase abgetrennt. Zu der organischen Phase werden 3,8 l n-Hexan gegeben und der Ansatz dreimal mit jeweils 1 345 ml 1N Schwefelsäure ausgerührt. Anschließend wird so oft mit je 1 345 ml Wasser ausgerührt, bis die wäßrige Phase pH 5 bis 6 hat. Dann wird das n-Hexan und die Restfeuchtigkeit bei 25°C Manteltemperatur und sich langsam verbesserndem Vakuum in eine tiefgekühlte Vorlage abdestilliert. Wenn 30 mbar erreicht sind, wird die Innentemperatur auf 80°C gesteigert. Anschließend wird noch 15 min bei 0,1 mbar/80°C andestilliert und unter Vakuum auf 20°C abgekühlt.
Ausbeute: 4 530 g
Gehalt nach GC: 99,3 %
= 89,1 %, bezogen auf 2-(2-Hydroxyethyl)-piperidin
= 94,5 %, bezogen auf Chlorameisensäure-sek.-butylester

### Beispiele 3 bis 20

0,1 mol Aminoalkohol werden in 100 ml Verdünnungsmittel gelöst oder suspendiert und 9,8 g (0,11 mol) 45 %ige Natronlauge sowie 12,9 ml Wasser zugesetzt und gerührt, bis alles in Lösung gegangen ist. Dann werden bei 20°C (Vergleichsfall) unter Kühlung bzw. erfindungsgemäß bei Siedetemperatur oder 80°C 0,09 mol Chlorameisensäureester unter gutem Rühren innerhalb von 10 min zugetropft. Es wird noch 30 min nachgerührt, wobei gegebenenfalls auf Raumtemperatur abgekühlt wird. Dann wird die wäßrige Phase abgetrennt, die organische Phase mit 1N Schwefelsäure und mit Wasser gewaschen und das Lösungsmittel am Rotationsverdampfer entfernt. Der Rückstand wird bis 50°C/0,1 mbar andestilliert.

Ein Maß für die Reinheit des Umsetzungsproduktes ist der Gehalt an diacyliertem Produkt [siehe dazu die folgende Tabelle: Verbindung der Formel (IV)]. Die Tabelle zeigt, daß die Selektivität der Umsetzung beim erfindungsgemäßen Verfahren deutlich besser ist, als bei niederen Temperaturen.

T a b e l l e

| Bsp. Nr. | Verbindung II | Verbindung III ($ClCO_2R^1$) $R^1$ = | Lösungsmittel | Temp. [°C] | Ausb. I [%, bezogen auf III] | Gehalt an Nebenprodukt IV* [%] |
|---|---|---|---|---|---|---|
| 3 | 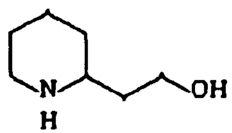 | $CH_3$ | Toluol | 20 | 94 | 4,5 |
| 4 | " | " | " | 80 | 93 | 1,5 |
| 5 | " | $C_2H_5$ | " | 20 | 91 | 2,7 |
| 6 | " | " | " | 80 | 93 | 1,4 |
| 7 | " | $-CH-C_2H_5$<br>\|<br>$CH_3$ | " | 20 | 85 | 2,7 |
| 8 | " | " | " | 80 | 88 | 0,7 |
| 9 | " | " | Cyclohexan | 20 | 78 | 4,5 |
| 10 | " | " | " | 80 | 90 | 0,9 |
| 11 | " | " | Methyl-tert.-butylether | 20 | 91 | 1,8 |

T a b e l l e (Fortsetzung)

| Bsp. Nr. | Verbindung II | Verbindung III ($ClCO_2R^1$) $R^1$ = | Lösungsmittel | Temp. [°C] | Ausb. I [%, bezogen auf III] | Gehalt an Nebenprodukt IV* [%] |
|---|---|---|---|---|---|---|
| 12 | 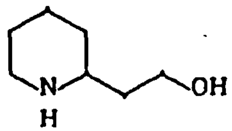 | 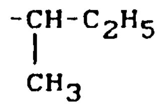 | Methyl-tert.-butylether | 55 | 88 | 0,7 |
| 13 | " | 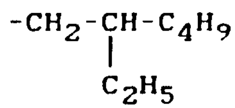 | Toluol | 20 | 86 | 2,9 |
| 14 | " | " | " | 80 | 86 | 1,7 |
| 15 | $H_2N-CH_2-CH_2-OH$ | 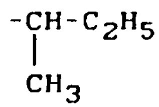 | " | 20 | 87 | 5,7 |
| 16 | " | " | " | 80 | 83 | <0,1 |
| 17 | 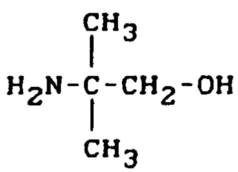 | " | " | 20 | 86 | 2,4 |
| 18 | " | " | " | 80 | 90 | 0,3 |

T a b e l l e (Fortsetzung)

| Bsp. Nr. | Verbindung II | Verbindung III ($ClCO_2R^1$) $R^1$ = | Lösungs-mittel | Temp. [°C] | Ausb. I [%, bezogen auf III] | Gehalt an Nebenprodukt IV* [%] |
|---|---|---|---|---|---|---|
| 19 | $H_3C-NH-CH_2-CH_2-CH_2-OH$ | $-CH_2-CH_2-CH(CH_3)_2$ | Toluol | 20 | 84 | 1,3 |
| 20 | " | " | " | 80 | 90 | <0,1 |

IV* = 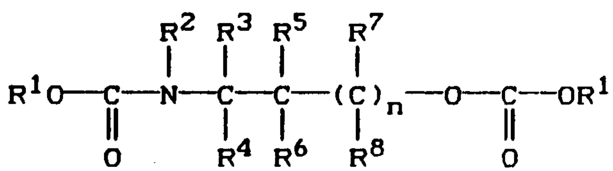

Beispiel 21

Es wird wie in Beispiel 1 beschrieben verfahren, mit dem Unterschied, daß 2-(2-Hydroxy-ethyl)-piperidin als wäßrige Lösung vorgelegt wird und die 45 %ige Natronlauge und der Chlorameisensäure-sec.-butylester simultan so zudosiert werden, daß ein pH-Wert von 10,5 bis 11,5 eingehalten wird. Es werden 414,1 g 2-(2-Hydroxyethyl)-piperidinyl-carbamidsäure-sec.-butylester mit einem gaschromatographisch bestimmten Gehalt von 99,0 % erhalten, entsprechend einer Ausbeute von 94,1 % der Theorie.

Vergleichsbeispiel A

In Analogie zu EP-A 289 842 werden 6,5 g (0,05 mol) 2-(2-Hydroxy-ethyl)-piperidin und 10 ml Triethylamin in 300 ml Tetrahydrofuran p.a. gelöst und bei -20°C mit 6,48 g (47,5 mmol) Chlorameisensäure-sec.-butylester versetzt. Dann wird 24 Stunden bei 20°C nachgerührt und mit Dichlormethan/Wasser extrahiert. Nach dem Trocknen der organischen Phase mit Magnesiumsulfat wird das Lösungsmittel im Vakuum am Rotationsverdampfer entfernt und der Rückstand bis 50°C/0,1 mbar andestilliert. Es werden 10,2 g eines Rohproduktes erhalten, welches nach gaschromatographischer Analyse 73 % 2-(2-Hydroxy-ethyl)-piperidinyl-carbamidsäure-sec.-butylester und 24 % 2-[2-(sec.-Butyloxy-carbonyl)-ethyl]-piperidinyl-carbamidsäure-sec.-butylester enthält.

Vergleichsbeispiel B

In Analogie zu J. Am. Chem. Soc. 69, 1384 (1947) werden 12,9 g (0,1 mol) 2-(2-Hydroxy-ethyl)-piperidin in 12,5 ml Wasser gelöst vorgelegt und bei 0 bis 3°C unter Eiskühlung gleichzeitig 25 ml 4N Natronlauge sowie 12,9 g (0,095 mol) Chlorameisensäure-sec.-butylester zugetropft. Es wird noch 30 min bei 0°C und 30 min bei Raumtemperatur nachgerührt. Der Ansatz wird mit Ether extrahiert, der Extrakt zweimal mit 1N Salzsäure und anschließend mit Wasser gewaschen, über Natriumsulfat getrocknet, am Rotationsverdampfer eingeengt und der Rückstand bei 50°C/0,1 mbar andestilliert. Es werden 18,3 g eines Rohproduktes erhalten, das nach gaschromatographischer Analyse 91,8 % 2-(2-Hydroxy-ethyl)-piperidinyl-carbamidsäure-sec.-butylester und 7,6 % 2-[2-(sec.-Butyloxy-carbonyl)-ethyl]-piperidinyl-carbamidsäure-sec.-butylester enthält, Auch hier ist also der Gehalt an diacyliertem Produkt wesentlich größer als beim erfindungsgemäßen Verfahren.

**Patentansprüche**

1. Verfahren zur Herstellung von N-(Hydroxyalkyl)-carbamidsäure-alkylestern der Formel (I)

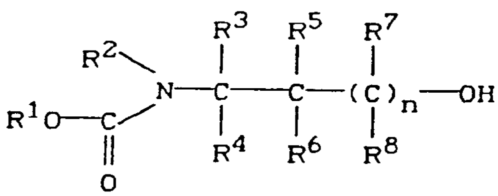

(I)

in welcher $R^1$ für geradkettiges oder verzweigtes Alkyl mit 1 bis 12 Kohlenstoffatomen steht und $R^2$ bis $R^8$ gleich oder verschieden sind und für Wasserstoff oder für Alkylreste mit 1 bis 8 Kohlenstoffatomen stehen oder $R^2$ und $R^3$ gemeinsam mit den Atomen, an welche sie gebunden sind, einen Ring mit 5 bis 7 Atomen, die mit $C_1$-$C_4$-Alkylresten substituiert sein können bilden, und n 0 oder 1 bedeutet,
durch Umsetzung von Aminoalkoholen der Formel (II)

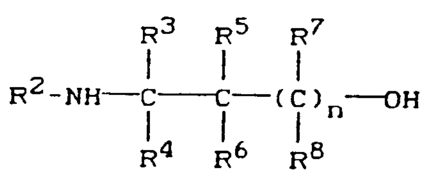

(II)

in welcher $R^2$ bis $R^8$ und n die oben angegebene Bedeutung haben,

mit Chlorameisensäureester der Formel (III)

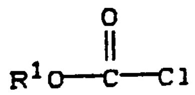

(III)

in welcher $R^1$ die oben angegebene Bedeutung hat,
dadurch gekennzeichnet, daß man die Umsetzung in Gegenwart von wäßrigen Alkalimetallhydroxid-Lösungen und gegebenenfalls in Anwesenheit eines organischen Lösungsmittels bei Temperaturen zwischen 30 und 110°C durchführt.

2. Verfahren gemäß Anspruch 1, dadurch gekennzeichnet, daß man im Temperaturbereich zwischen 50 und 90°C arbeitet.

3. Verfahren gemäß Anspruch 1 und 2, dadurch gekennzeichnet, daß als Alkalimetallhydroxid-Lösungen wäßrige Lösungen von Natriumhydroxid eingesetzt werden.

4. Verfahren gemäß Anspruch 1 bis 3, dadurch gekennzeichnet, daß pro Mol Aminoalkohol der Formel (II) 0,6 bis 1,3 Mol Chlorameisensäureester der Formel (III) eingesetzt werden,

5. Verfahren gemäß Anspruch 1 bis 3, dadurch gekennzeichnet, daß pro Mol Aminoalkohol der Formel (II) 0,8 bis 1,0 Mol Chlorameisensäureester der Formel (III) eingesetzt werden.

6. Verfahren gemäß Anspruch 1 bis 5, dadurch gekennzeichnet, daß pro Mol Chlorameisensäureester der Formel (III) 0,9 bis 2,0 Mol Alkalimetallhydroxid eingesetzt werden.

7. Verfahren gemäß Anspruch 1 bis 6, dadurch gekennzeichnet, daß pro Mol Chlorameisensäureester der Formel (III) 1,0 bis 1,2 Mol Alkalimetallhydroxid eingesetzt werden.

8. Verfahren gemäß Anspruch 1 bis 7 zur Herstellung von Verbindungen der allgemeinen Formel (I), in welcher
die Reste $R^2$ bis $R^8$ für Wasserstoff, Methyl oder Ethyl stehen oder die Reste $R^2$ und $R^3$, gemeinsam mit den Atomen, an welche sie gebunden sind, einen unsubstituierten Ring mit 5 bis 7 Atomen stehen und n 0 oder 1 bedeutet.

9. Verfahren gemäß Anspruch 1 bis 7 zur Herstellung von Verbindungen der allgemeinen Formel (I), in welcher
$R^2$ für Methyl steht, $R^3$ bis $R^8$ für Wasserstoff stehen oder wobei $R^2$ und $R^3$ gemeinsam mit den Atomen, an welche sie gebunden sind, den Piperidin-Ring bedeuten und n für 1 steht.

## Claims

1. Process for the preparation of alkyl N-(hydroxyalkyl)-carbamates of the formula (I)

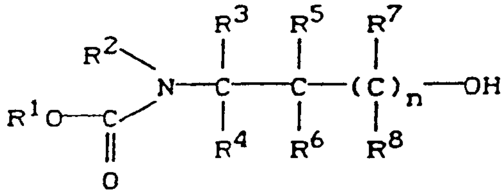

(I)

in which $R^1$ represents straight-chain or branched alkyl having 1 to 12 carbon atoms and $R^2$ to $R^8$ are identical or different and represent hydrogen or alkyl radicals having 1 to 8 carbon atoms, or $R^2$ and $R^3$ together with the atoms to which they are bonded form a ring having 5 to 7 atoms which may be substituted by $C_1$-$C_4$-alkyl radicals, and n denotes 0 or 1,

by reacting aminoalcohols of the formula (II)

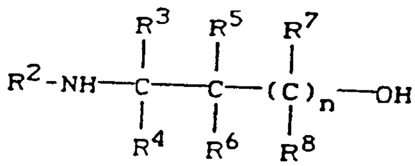

(II)

in which $R^2$ to $R^8$ and n have the abovementioned meaning,
with chloroformates of the formula (III)

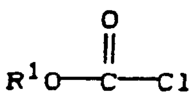

(III)

in which $R^1$ has the abovementioned meaning,
characterized in that the reaction is carried out in the presence of aqueous alkali metal hydroxide solutions and, if appropriate, in the presence of an organic solvent at temperatures between 30 and 110°C.

2. Process according to Claim 1, characterized in that the process is carried out in the temperature range between 50 and 90°C.

3. Process according to Claims 1 and 2, characterized in that the aqueous solutions of sodium hydroxide are employed as alkali metal hydroxide solutions.

4. Process according to Claims 1 to 3, characterized in that 0.6 to 1.3 mol of chloroformate of the formula (III) are employed per mole of aminoalcohol of the formula (II).

5. Process according to Claims 1 to 3, characterized in that 0.8 to 1.0 mol of chloroformate of the formula (III) are employed per mole of aminoalcohol of the formula (II).

6. Process according to Claims 1 to 5, characterized in that 0.9 to 2.0 mol of alkali metal hydroxide are employed per mole of chloroformate of the formula (III).

7. Process according to Claims 1 to 6, characterized in that 1.0 to 1.2 mol of alkali metal hydroxide are employed per mole of chloroformate of the formula (III).

8. Process according to Claims 1 to 7 for the preparation of compounds of the general formula (I) in which the radicals $R^2$ to $R^8$ represent hydrogen, methyl or ethyl or the radicals $R^2$ and $R^3$ together with the atoms to which they are bonded form an unsubstituted ring having 5 to 7 atoms, and n denotes 0 or 1.

9. Process according to Claims 1 to 7 for the preparation of compounds of the general formula (I) in which $R^2$ represents methyl, $R^3$ to $R^8$ represent hydrogen, or where $R^2$ and $R^3$ together with the atoms to which they are bonded, denote the piperidine ring, and n represents 1.

## Revendications

1. Procédé pour fabriquer des esters alkyliques d'acides N-(hydroxyalkyl)-carbamiques répondant à la formule (I)

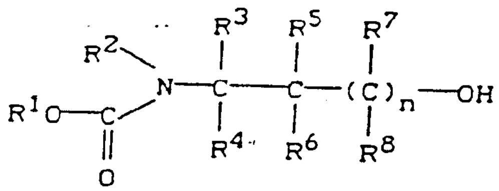

(I)

dans laquelle $R^1$ signifie un groupe alkyle à chaîne linéaire ou ramifiée avec 1 à 12 atomes de carbone et $R^2$ à $R^8$ sont identiques ou différents et signifient l'hydrogène ou un reste alkyle avec 1 à 8 atomes de carbone ou $R^2$ et $R^3$ forment, ensemble avec les atomes sur lesquels ils sont fixés un cycle avec 5 à 7 atomes, et n signifie 0 ou 1,
en faisant réagir des aminoalcools de formule (II)

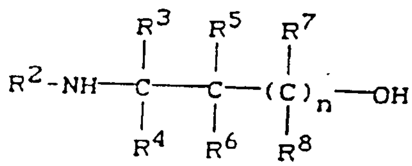

(II)

dans laquelle $R^2$ à $R^8$ et n possèdent la signification décrite ci-dessus, avec de l'ester d'acide chloroformique de formule (III)

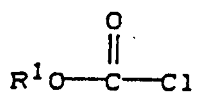

(III)

dans laquelle $R^1$ possède la signification décrite ci-dessus,
procédé caractérisé en ce que l'on effectue la réaction en présence de solutions aqueuses d'hydroxydes de métaux alcalins et éventuellement en présence d'un solvant organique à des températures de 30 à 110°C.

2. Procédé selon la revendication 1, caractérisé en ce que l'on travaille à des températures de 50 à 90°C.

3. Procédé selon les revendications 1 et 2, caractérisé en ce que l'on utilise comme solutions d'hydroxyde de métal alcalin des solutions aqueuses d'hydroxyde de sodium.

4. Procédé selon les revendications 1 à 3, caractérisé en ce que l'on utilise 0,6 à 1,3 mole d'ester d'acide chloroformique de formule (III) par mole d'aminoalcool de formule (II).

5. Procédé selon les revendications 1 à 3, caractérisé en ce que l'on utilise par mole d'aminoalcool de formule (II) 0,8 à 1,0 mole d'ester d'acide chloroformique de formule (III).

6. Procédé selon les revendications 1 à 5, caractérisé en ce que l'on utilise par mole d'ester d'acide chloroformique de formule (III) 0,9 à 2,0 moles d'hydroxyde de métal alcalin.

7. Procédé selon les revendications 1 à 6, caractérisé en ce que l'on utilise par mole d'ester d'acide chloroformique de formule (III) 1,0 à 1,2 mole d'hydroxyde de métal alcalin.

8. Procédé selon les revendications 1 à 7 pour fabriquer des composés de formule générale (I) dans lesquels les restes $R^2$ à $R^8$ signifient l'hydrogène, un groupe méthyle ou éthyle ou les restes $R^2$ et $R^3$ signifient ensemble avec les atomes sur lesquels ils sont fixés un cycle non substitué avec 5 à 7 atomes et n signifie 0 ou 1.

9. Procédé selon les revendications 1 à 7 pour fabriquer les composés de formule générale (I), dans lesquels $R^2$ signifie un groupe méthyle, $R^3$ à $R^8$ signifient l'hydrogène, ou $R^2$ et $R^3$ signifient ensemble avec les atomes sur lesquels ils sont fixés le cycle pipéridine et n signifie 1.